# DEMANDE DE BREVET EUROPEEN

(11) **EP 1 647 265 A1**
(43) Date de publication de la demande: **19.04.2006**
(21) Numéro de dépôt: 05292146.7
(22) Date de dépôt: 13.10.2005
(51) Int. Cl.: A61K 8/40, A61Q 5/06, A61K 8/90

(54) **Utilisation pour le traitement cosmétique des matières kératiniques de compositions non collantes à base de monomères électrophiles et de polymères non siliconés**

(30) Priorité: 13.10.2004 FR 0410807
(71) Demandeur: L'OREAL, 75008 Paris (FR)
(72) Inventeur: Vic, Gabin, 60280 Venette (FR); Livoreil, Aude, 75006 Paris (FR); Brun, Gaelle, 75015 Paris (FR); Gourlaouen, Luc, 92600 Asnieres (FR); Giroud, Franck, 92110 Clichy (FR); Rollat-Corvol, Isabelle, 75017 Paris (FR)
(74) Mandataire: Casalonga, Axel

(57) **Abrégé**

La présente demande a pour objet l'utilisation pour le traitement cosmétique des matières kératiniques, et notamment des fibres kératiniques telles que les cheveux, d'une composition comprenant, dans un milieu cosmétiquement acceptable, au moins un monomère électrophile, et au moins un polymère non siliconé, le polymère non siliconé étant choisi de façon à ce que la composition donne après séchage un film présentant une force maximale de décollement supérieure à 1 Newton.

## Description

La présente invention concerne l'utilisation pour le traitement cosmétique des matières kératiniques, et notamment des fibres kératiniques telles que les cheveux, de compositions non collantes à base de monomères polymérisables in situ, d'un milieu cosmétiquement acceptable, et de polymères non siliconés particuliers, ainsi que le procédé de traitement cosmétique correspondant.

Par "matières kératiniques", on entend de préférence les fibres kératiniques, et encore de préférence les cheveux.

On utilise généralement des compositions cosmétiques à base de silicones ou de polymères ayant une forte affinité pour les matières kératiniques, en particulier les cheveux, en vue de modifier leurs propriétés superficielles, notamment pour les conditionner et leur apporter de la brillance.

Il est généralement nécessaire de renouveler ces traitements dans la mesure où les agents de conditionnement ont tendance à s'éliminer, notamment aux shampoings.

Il est théoriquement possible d'accroître la rémanence du dépôt de polymères en effectuant directement une polymérisation radicalaire de certains monomères au niveau des cheveux.

Toutefois, les traitements ainsi obtenus sont inacceptables au point de vue cosmétique. On constate généralement une forte dégradation de la fibre liée probablement aux amorceurs de polymérisation et les cheveux traités sont difficilement démêlables, si bien qu'aujourd'hui, on cherche à obtenir des compositions cosmétiques permettant de conférer de la douceur et une brillance durable à la chevelure.

En outre, les produits cosmétiques destinés aux traitements des cheveux et notamment ceux permettant d'obtenir des effets de forme et de coiffure utilisent généralement des compositions formant sur les cheveux un film collant ou « tackant » (présentant une adhésion de surface). De façon générale, le toucher des cheveux ainsi traités est particulièrement rêche et désagréable. Lorsque l'utilisateur passe la main sur les cheveux, une partie des polymères peut se déposer sur les doigts. Ce phénomène de transfert laisse une impression de cheveux sales. En outre, ce revêtement tackant s'élimine immédiatement lors du lavage des cheveux. Il est donc nécessaire de réappliquer du produit après chaque shampooing.

Il existe donc un besoin de réaliser des compositions qui n'adhèrent pas aux doigts après application sur les cheveux, qui ne poudrent pas et qui confèrent aux cheveux de bonnes propriétés cosmétiques, rémanentes aux lavages

La demanderesse vient de découvrir de manière surprenante qu'il était possible d'atteindre les objectifs mentionnés ci-dessus, en mettant en oeuvre un mélange de monomères électrophiles, tels qu'ils sont décrits dans la demande FR 2840208, d'un milieu cosmétiquement acceptable et d'un polymère non siliconé particulier.

La demanderesse a plus particulièrement constaté qu'en appliquant une composition à base de tels monomères, d'un milieu cosmétiquement acceptable et d'un polymère non siliconé particulier sur les fibres kératiniques, il se forme in situ un revêtement lubrifiant, brillant et doux rémanent.

Par ailleurs, la demanderesse a constaté de façon surprenante que les cheveux restaient parfaitement individualisés et pouvaient être coiffés sans problème, et que le conditionnement et la brillance de la fibre étaient rémanents aux shampooings.

L'invention a donc pour premier objet l'utilisation pour le traitement cosmétique des matières kératiniques, et notamment des fibres kératiniques telles que les cheveux, d'une composition comprenant, dans un milieu cosmétiquement acceptable, au moins un monomère électrophile, et au moins un polymère non siliconé, le polymère étant choisi de façon à ce que la composition donne après séchage un film présentant une force maximale de décollement supérieure à 1 Newton.

L'invention a également pour objet un procédé mettant en oeuvre une composition utilisée dans le cadre de ce traitement.

Elle a aussi pour objet un kit comprenant une première composition contenant au moins un monomère électrophile (présent à des teneurs pouvant être comprise entre 0,5 et 50% du poids de la première composition) et éventuellement au moins un inhibiteur de polymérisation anionique et/ou radicalaire (présent à des teneurs pouvant être comprise entre 10 ppm et 5% du poids de la première composition), ainsi qu'une deuxième composition comprenant dans un milieu cosmétiquement acceptable au moins un polymère non siliconé tel que défini ci-dessus (présent à des teneurs pouvant être comprise entre 0,001 et 5% du poids de la deuxième composition).

Elle a enfin pour objet une composition cosmétique comprenant au moins un monomère électrophile et au moins un polymère fixant non siliconé.

D'autres objets de l'invention apparaîtront à la lecture de la description et des exemples qui suivent.

Selon la présente invention, on entend par « force maximale de décollement » la force maximale de traction, mesurée à l'aide d'un extensomètre, nécessaire pour décoller les surfaces de 38 mm ² respectives de deux supports (A) et (B) rigides, inertes, non-absorbants, placés en regard l'un de l'autre ; lesdites surfaces étant enduites préalablement par ladite composition à raison de 519 µg/mm², séchées pendant 24 heures à 22°C sous une humidité relative de 50%, puis soumis pendant 20 secondes à une compression de 3 Newtons et enfin soumis pendant 30 secondes à une traction de vitesse 20 mm/minute.

On peut utiliser pour cette mesure un extensomètre, par exemple un appareil du type LLOYD modèle LR5K.

Les supports, rigides, inertes et non absorbants peuvent être choisis parmi ceux constitués de polyéthylène, polypropylène, alliages métalliques, et plus préférentiellement de verre.

On utilise de préférence à titre de supports une paire de plots constitués d'un disque de verre surmontant une tige nécessaire pour l'accroche par les mors de l'extensomètre. ledit disque est de préference de la taille du plot et fixé à celui-ci par une colle du type ARALDITE®. La composition de coiffage à tester est répartie de la façon la plus uniforme possible sur la surface de chaque disque de verre et mise à sécher de façon à ce que la surface reste plane.

On détermine le profil de décollement en mesurant Fmax correspondant à la force de traction, mesurée à l'aide de l'extensomètre, nécessaire pour décoller les surfaces respectives des deux disques. On procédera de préférence selon le protocole suivant : on prépare 6 paires de plots. On réalise pour chaque paire de plots, un test de décollement selon le mode opératoire indiqué ci-dessus pour le test 1. On sélectionne les résultats obtenus sur les 6 profils de décollement effectués en excluant pour chaque paire de plots, les cas où les matériaux coiffants se sont décollés de l'un de plots de la paire. On détermine pour chaque profil de décollement restant, la Fmax. On réalise la moyenne de ces mesures.

De préférence, le polymère non siliconé est choisi de façon à conférer à la composition définie ci-dessus une énergie de séparation Es(M/V) inférieure à 300 µJ.

Selon la présente invention, on entend par «Es(M/V)» : l'énergie fournie par l'extensomètre pour effectuer la « séparation » des surfaces respectives de 38 mm², de deux supports (C) et (D), rigides, inertes, non absorbants et placés en regard l'un de l'autre ; l'un desdits supports étant constitués de verre poli et l'autre desdits supports étant de nature identique à celle des supports (A) et (B) définis ci-dessus et dont la surface est enduite de la composition définie ci-dessus à raison de 519 µg/mm² sur le support, séchée pendant 24 heures à 22°C sous une humidité relative de 50%, puis soumis pendant 20 secondes à une compression de 3 Newtons et enfin soumises pendant 30 secondes à une traction de vitesse 30mm/minute.

Cette énergie fournie par l'extensomètre est le travail déterminé par l'intégrale de F(x)dx entre Xsl+0,05 et Xs2 ; où F(x) est la force nécessaire pour produire un déplacement (x) ; Xs1 est la déplacement (exprimé en millimètres) produit par la force de traction maximale ; Xs2 est le déplacement (exprimé en millimètres) produit par la force de traction permettant la séparation totale des deux surfaces.

Mode opératoire relatif à la moyenne de Es(M/V) : on détermine l'énergie fournie par l'extensomètre pour effectuer la « séparation » des surfaces respectives de deux supports et de 38 mm², rigides et inertes non absorbants et placés en regard l'un de l'autre ; l'un desdits supports étant constitué de verre poli et l'autre desdits supports étant de nature identique à celle des supports définis ci-dessus et dont la surface est enduite et traitée dans les mêmes conditions que celles du premier mode opératoire décrit ci-dessus et en utilisant un extensomètre du même type que précédemment. De façon préférentielle, on procédera selon le protocole suivant : on prépare 6 paires de plots. On réalise pour chaque paire de plots, un test de décollement selon le mode opératoire indiqué ci-dessus. On sélectionne les résultats obtenus sur les 6 profils de décollement effectués en excluant pour chaque paire de plots, les cas où les matériaux coiffants se sont décollés de l'un des plots de la paire. On détermine, pour chaque profil de décollement restant, le Es(M/V). On réalise la moyenne de ces mesures.

Le polymère non siliconé peut être dendritique, linéaire ou ramifié, peigne, bloc ou étoile. Il peut présenter un ou plusieurs types de motifs de répétition et peut ainsi être choisi parmi les homopolymères et les copolymères statistiques, alternés ou séquencés. Par polymère on entend un composé comportant au moins 5 motifs de répétition enchaînés par des liaisons covalentes. il peut notamment être choisi parmi les copolymères linéaires séquencés amphiphiles.

Par « non siliconé », on entend au sens de la présente invention un polymère qui ne contient pas d'enchaînement -SiOSi-.

Le squelette principal du polymère comprend des atomes de C et de H, et peut être interrompu par un ou plusieurs hétéroatomes tels que O, N, P et S, et peut comporter des fonctions en bout de chaîne ou latérales.

Les copolymères blocs peuvent être di-, tri- ou multiblocs, linéaires ou en étoiles. Ils peuvent être hydrosolubles, hydrodispersibles, ou liposolubles.

Dans le cas où ils se solubilisent ou se dispersent spontanément ou par neutralisation dans l'eau, ils peuvent être constitués d'au moins un bloc à caractère hydrophile et d'au moins un bloc à caractère hydrophile de composition chimique différente du précédent, ou bien ils peuvent être constitués d'au moins un bloc à caractère hydrophile et d'au moins un bloc à caractère hydrophobe. Ils sont de caractère anionique, cationique, non ionique ou amphotère.

Dans le cas où ils se solubilisent ou se dispersent spontanément dans un milieu anhydre, ils peuvent être constitués d'au moins un bloc à caractère hydrophobe et d'au moins un bloc à caractère hydrophobe de composition chimique différente du précédent, ou bien ils peuvent être constitués d'au moins un bloc à caractère hydrophobe et d'au moins un bloc à caractère hydrophile. Ils sont de caractère anionique, cationique, non ionique ou amphotère.

On entend par copolymère à blocs hydrosoluble ou hydrodispersible, ou liposoluble, un copolymère qui, à la concentration de 0,1% de matière active dans l'eau ou dans un solvant anhydre à 25°C, conduit spontanément ou après neutralisation à l'aide d'un acide ou d'une base à une solution ou suspension macroscopiquement homogène, transparente ou translucide, c'est-à-dire ayant une valeur de transmittance à une longueur d'onde de 500 nm à travers un échantillon de 1 cm d'épaisseur d'au moins 70%, de préférence 80%.

On entend par bloc à caractère hydrophile un bloc constitué d'au moins 75% en poids de monomères hydrosolubles et/ou hydrosolubilisables par neutralisation. Le bloc hydrophile peut ainsi contenir jusqu'à 25% en moles d'un ou plusieurs monomères non hydrosolubles. Cette proportion est de préférence égale à 10% en moles et, idéalement, inférieure ou égale à 5% en moles.

On entend par bloc à caractère hydrophobe un bloc constitué d'au moins 75% en poids de monomères non hydrosolubles. Le bloc hydrophile peut ainsi contenir jusqu'à 25% en moles d'un ou plusieurs monomères hydrosolubles. Cette proportion est de préférence égale à 10% en moles et, idéalement, inférieure ou égale à 5% en moles.

A titre d'exemples de monomères utilisables dans les polymères de l'invention, on peut citer ceux décrits dans la demande FR 2 840 205.

Les monomères hydrosolubles formant le ou les blocs hydrophiles des copolymères séquencés pouvant être utilisés dans la présente invention peuvent être de nature anionique, non-ionique ou cationique et peuvent être utilisés seuls ou sous forme de mélange contenant deux ou plusieurs monomères différents.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropanesulfonique, l'acide styrènesulfonique, l'acide vinylsulfonique et l'acide vinylphosphonique.

Les monomères hydrosolubles non-ioniques englobent, entre autres, l'acrylamide, les acrylamides N-alkylés en C1-C6 ou N,N-dialkylés en C1-C3, l'acrylate de polyéthylèneglycol, le méthacrylate de polyéthylèneglycol, le N-vinylacétamide, le N-méthyl-N-vinlacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate de d'hydroxpropyle.

Enfin, les monomères hydrosolubles cationiques englobent par exemple le chlorure de diméthyldiallylammonium, le chlorure de méthylvinylimidazolium, la 2-vinylpyridine, la 4-vinylpyridine, la 2-méthyl-5-vinylpyridine, les halogénures de N-(alkyle en C1-C4)-4-vinylpyridinium tels que l'iodure de N-méthyl-4-vinylpyridinium, la vinylamine, les monomères de formule

H₂C=CR'₁-CO-X₂

dans laquelle
R'₁ représente un atome d'hydrogène ou un groupe méthyle,
X₂ représente un groupe hydrocarboné linéaire ou ramifié en C1-C6 portant au moins une fonction amine primaire, secondaire ou tertiaire ou au moins un atome d'azote quaternaire, ou un groupe de formule NHR'₂ ou de formule NR'₂R'₃ où R'₂ et R'₃ représentent indépendamment l'un de l'autre chacun un groupe hydrocarboné en C1-C6, linéaire ou ramifié, portant au moins une fonction amine primaire, secondaire ou tertiaire ou au moins un atome d'azote quaternaire.

Les monomères insolubles dans l'eau (non hydrosolubles) formant le ou les blocs hydrophobes des copolymères séquencés sont choisis de préférence parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C1-C10, d'aryle en C6-C10 ou d'aralkyle en C6-C10 et les méthacrylates d'alkyle en en C1-C10, d'aryle en C6-C10 ou d'aralkyle en C6-C10, comme par exemple les (méth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-OR" et les allyléthers de formule CH₂=CH-CH₂-O-R", où R" représente un groupe alkyle en C1-C6, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroalkylates d'alkyle.

Le polymère non siliconé selon l'invention est tout particulièrement choisi parmi les polymères fixants non siliconés.

Les polymères fixants non siliconés convenant dans l'invention sont notamment choisis parmi les polymères fixants non siliconés cationiques, anioniques, amphotères, non ioniques et leurs mélanges.

Les polymères fixants non siliconés cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 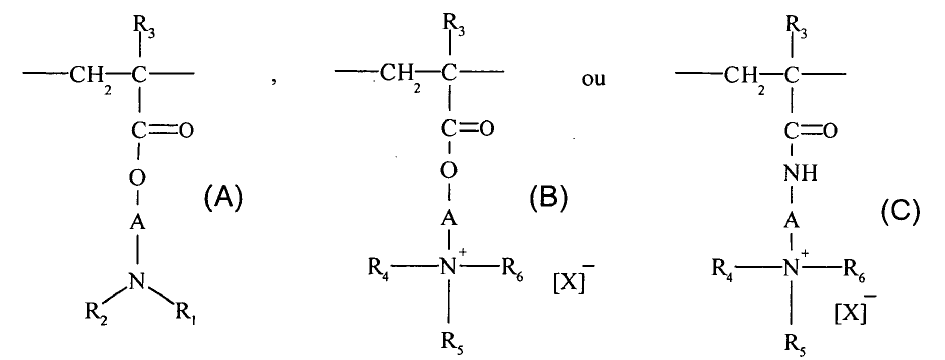 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT® " par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français Nos 2 077 143 et 2 393 573,
   - les polymères à chaîne grasse et à motif vinylpyrrolidone, tels que les produits vendus sous la dénomination Stylène W20 et Stylène W10 par la société ISP,
   - les terpolymères méthacrylate de diméthylaminoéthyle/ vinylcaprolactame/vinylpyrrolidone tels que le produit commercialisé sous la dénomination GAFFIX® VC 713 par la société ISP, et
   - les copolymères vinylpyrrolidone/méthacrylamide de diméthylaminopropyle quaternisé tels que notamment le produit commercialisé sous la dénomination "GAFQUAT® HS 100" par la société ISP ;
(2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.
   Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants non siliconés anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 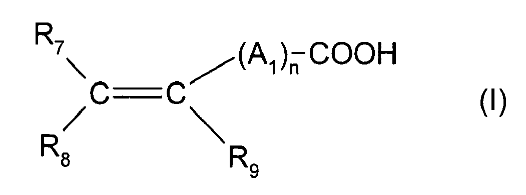 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants non siliconés anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁₋C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
      les fonctions anhydrides de ces copolymères étant éventuellement monoestérifiées ou monoamidifiées.
      Ces polymères sont par exemple décrits dans les brevets français n^{os} 2 350 384 et 2 357 241 de la demanderesse.
E) Les polyacrylamides comportant des groupements carboxylates.
   Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan® 500 et Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

Comme autre polymère fixant non siliconé anionique utilisable selon l'invention, on peut citer le polymère anionique séquencé branché vendu sous la dénomination FIXATE G100 par la société NOVEON.

Selon l'invention, les polymères fixants non siliconés anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination FIXATE G100 par la société NOVEON.

Parmi les polymères fixants non siliconés anioniques cités ci-dessus, on préfère plus particulièrement utiliser dans le cadre de la présente invention les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination FIXATE G100 par la société NOVEON.

Les polymères fixants non siliconés amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α, β-dicarboxylique dont l'un des groupements carboxyliques a été amené à réagir avec une polyamine comportant un ou plusieurs groupements amine primaire ou secondaire.

Les polymères fixants non siliconés amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
      Les comonomères basiques préférés sont des méthacrylates d'aminoéthyle, de butylaminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethyl methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale : 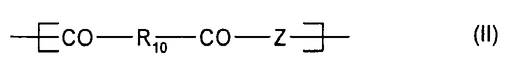 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 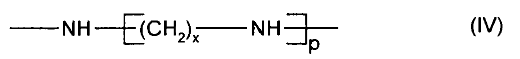 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 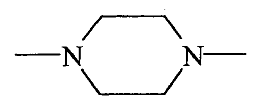
   c) dans les proportions de 0 à 20 % en moles, le groupe -NH-(CH₂)₆-NH- dérivant de l'hexaméthylènediamine,
      ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
      Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 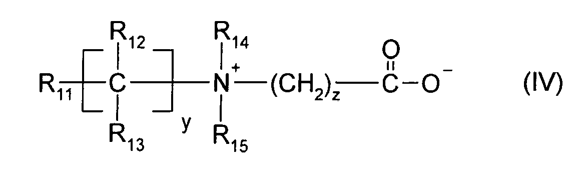 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tels que le produit vendu sous la dénomination DIAFORMER Z301 par la société SANDOZ.
(5) les polymères dérivés du chitosane comportant des motifs monomères répondant aux formules suivantes : 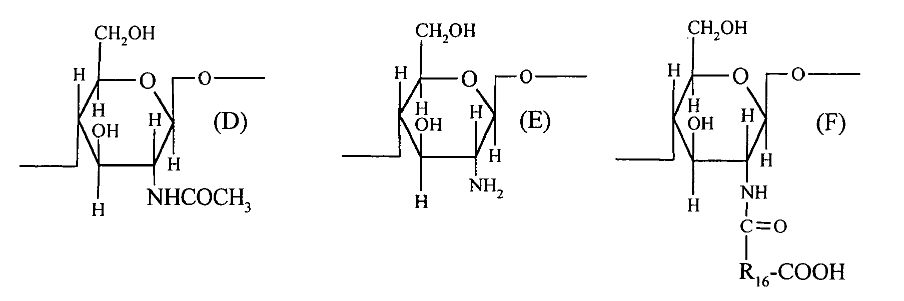 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 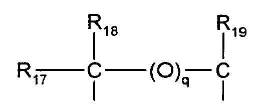 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 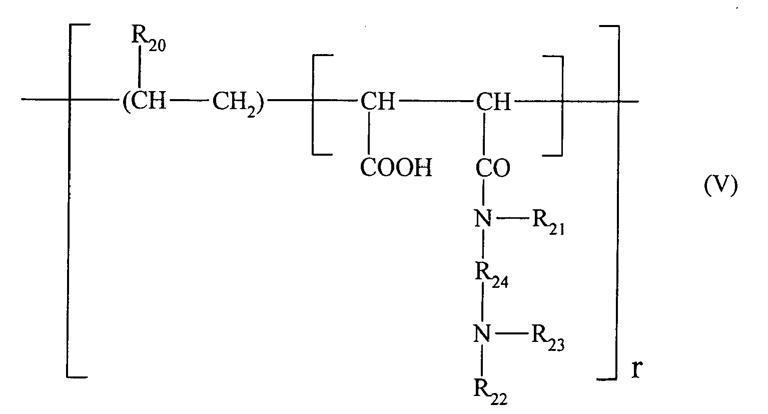 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂₋CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 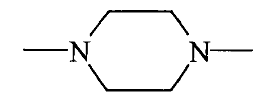 et X désigne le symbole E ou E', E ou E' identiques ou différents désignent un' groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 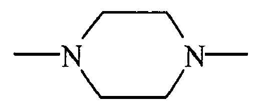 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
   (9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.
      Parmi les polymères fixants non siliconés amphotères décrits ci-dessus, les plus particulièrement préférés selon l'invention sont ceux de la famille (3) tels que les copolymères dont la dénomination CTFA est Octylacrylamide/ acrylates/butylamino-ethylmethacrylate copolymer, tels que les produits vendus sous les dénominations AMPHOMER® , AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER® Z301 par la société SANDOZ.

Les polymères fixants non siliconés non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyle des polymères non ioniques mentionnés ci-dessus ont, de préférence, de 1 à 6 atomes de carbone.

On peut également utiliser comme polymères fixants non siliconés, des polyuréthanes fonctionnalisés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer le produit commercialisé sous la dénomination LUVISET PUR® par la société BASF.

Le ou les polymères non siliconés peuvent être présents dans la composition à des teneurs comprises entre 0,05 et 99% en poids, de préférence entre 0,1 et 95%, et encore de préférence entre 0,2 et 30% en poids du poids total de la composition.

Par monomère électrophile, on entend un monomère capable de polymériser par polymérisation anionique en présence d'un agent nucléophile tel que par exemple, les ions hydroxyles (OH-) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
- les dérivés benzylidene malononitrile (A), le 2-(4-chloro-benzylidene)-malononitrile (A1) le 2-cyano-3-phényl acrylate d'éthyle (B), le 2-cyano-3-(4-chloro-phényl) acrylate d'éthyle (B1) décrits dans Sayyah, *J. Polymer Research,* 2000, p97 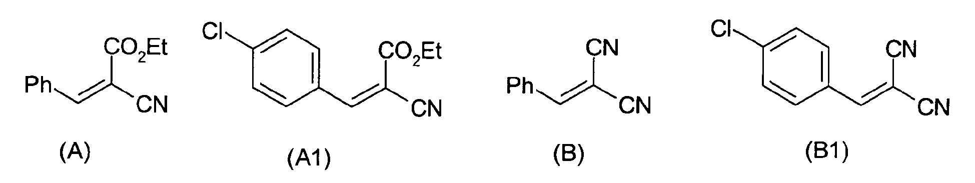
- les dérivés de méthylidenemalonates comme :
   - le 2-méthylene-malonate de diéthyle (C) par Hopff, *Makromoleculare Chemie,* 1961, p95, De Keyser, *J. Pharm. Sci,* 1991, p67 et Klemarczyk, *Polymer,* 1998, p173 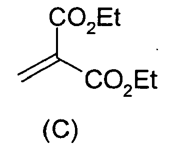
   - le 2-éthoxycarbonylméthyleneoxycarbonyl acrylate d'éthyle (D) par Breton, *Biomaterials,* 1998, p271 et Couvreur, *Pharmaceutical Research,* 1994, p1270. 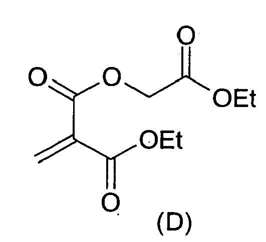
- les dérivés itaconate et itaconimide comme :
   - l'itaconate de diméthyle (E) par Bachrach, *European Polymer Journal,* 1976, p563 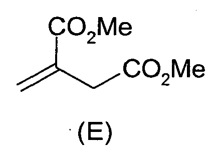
   - N-butyl itaconimide (F), N-(4-tolyl) itaconimide (G), N-(2-ethylphenyl) itaconimide (H), N-(2,6-diethylphenyl) itaconimide (I) par Wanatabe, *J.Polymer Science : Part A :Polymer chemistry,* 1994, p2073 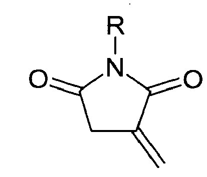 R= Bu (F), 4-tolyl (G), 2-ehylphenyl (H), 2,6-diethyphenyl (1)
- les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O), par Gipstein, *J. Org. Chem,* 1980, p1486 et
- les dérivés 1,1-bis-(methylsulfonyl)ethylene (P), 1-acetyl-1-methyl sulfonyl ethylene (Q), α-(methylsulfonyl) vinyl sulfonate de methyle (R), α-methylsulfonylacrylonitrile (S) par Shearer, US patent US2748050. 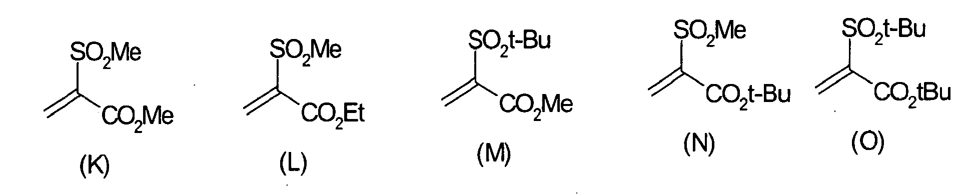 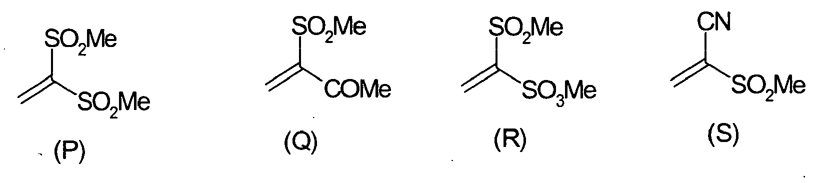
- les dérivés méthyl vinyl sulfone (T) et phényl vinyl sulfone (U) par Boor, *J.Polymer Science,* 1971, p249 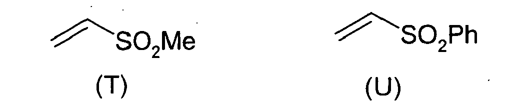
- le dérivé phényl vinyl sulfoxide (V) par Kanga, *Polymer preprints (ACS, Divison of Polymer Chemistry),* 1987, p322 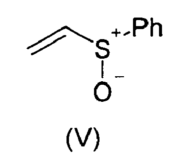
- le dérivé 3-methyl-N-(phenylsulfonyl)-1-aza-1,3-butadiene (W) par Bonner, *Polymer Bulletin,* 1992, p517 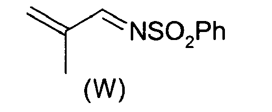
- les dérivés acrylates et acrylamides comme :
   - N-propyl-N-(3-triisopropoxysilylpropyl)acrylamide (X) et N-propyl-N-(3-triethoxysilylpropyl)acrylamide (Y) par Kobayashi, *Journal of Polymer Science, Part A: Polymer Chemistry,* 2005, p2754. 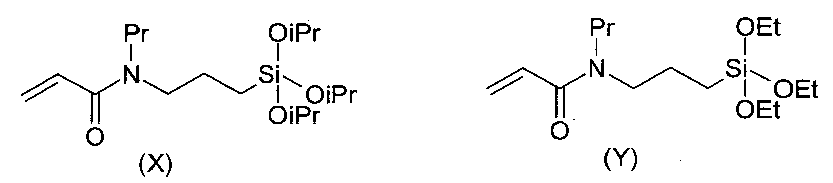
   - 2-hydroxyethyl acrylate (Z) et 2-hydroxyethyl méthacrylate (AA) par Rozenberg, *International Journal of Plastics Technology,* 2003, p17 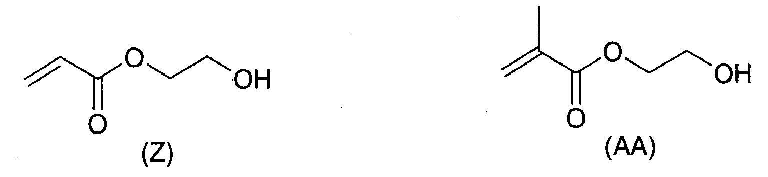
   - N-butyl acrylate (AB) par Schmitt, *Macromolecules,* 2001, p2115
   - Tert-butyl acrylate (AC) par Ishizone, *Macromolecules,* 1999, p955.

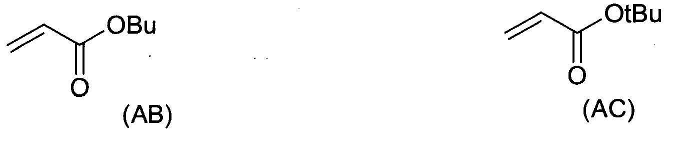

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (I) : 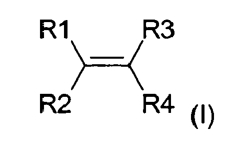 dans laquelle :
R1 et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR,
   - OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, - OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère, R' désignant un radical alkyle en C₁-C₁₀, ce polymère pouvant être obtenu par polymérisation radicalaire, par polycondensation ou par ouverture de cycle.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)n-(CF₂)m-CF₃ ou -(CH₂)n-(CF₂)m-CHF₂ avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoïques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (II) : 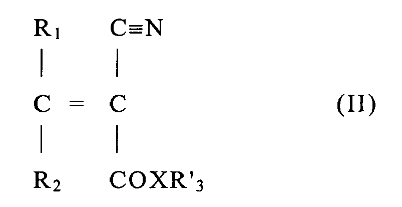 X désignant NH,S,O,
R₁ et R₂ ayant les mêmes significations que précédemment,
R'₃ pouvant désigner un atome d'hydrogène ou un radical R tel que défini pour la formule (I).

De préférence, X désigne O.

A titre de composés de formule (II), on peut citer les monomères :
a) appartenant à la famille des 2-cyanoacrylates de polyfluoroalkyle en C₁-C₂₀ tels que :
   l'ester 2,2,3,3-tétrafluoropropylique de l'acide 2-cyano-2-propénoïque de formule : 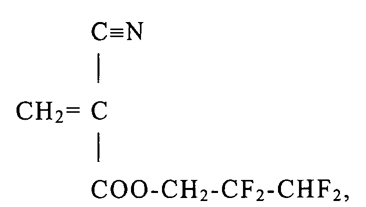
   ou encore l'ester 2,2,2-trifluoroéthylique de l'acide 2-cyano-2-propénoïque de formule : 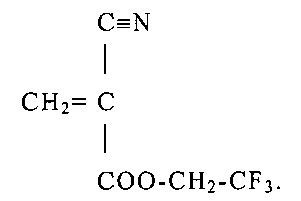
b) les cyanoacrylates d'alkyle en C₁-C₁₀ ou d'(alcoxy en C₁₋C₄)(alkyle en C₁-C₁₀).
   On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères les plus particulièrement préférés sont ceux de formule III et leurs mélanges : 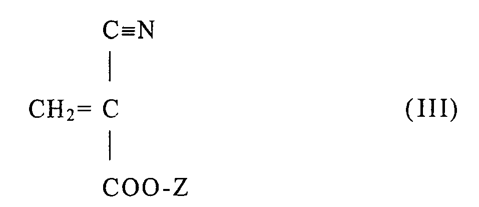 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Par milieu cosmétiquement acceptable, on entend un milieu compatible avec les matières kératiniques telles que les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique, l'alcool béhénylique ; les acides gras en C₁₀-C₃₀ tels que l'acide laurique, l'acide stéarique ; les amides gras en C₁₀-C₃₀ tels que la diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 105 Pa (760mm de Hg).

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, tel que, par exemple, des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des gaz propulseurs, des épaississants minéraux ou organiques tels que le benzylidène sorbitol, les N acylaminoacides. Ces agents peuvent être éventuellement encapsulés. La capsule peut être de type polycyanoacrylate.

Le procédé de traitement des cheveux conforme à l'invention consiste à appliquer la composition décrite ci-dessus sur les matières kératiniques, et en particulier en présence d'un agent nucléophile avec ou sans chauffage.

De préférence, cet agent nucléophile est l'eau. Cette eau peut être apportée par une humidification préalable.

Il est également possible, afin de moduler la cinétique de réaction, de préalablement humidifier les matières kératiniques à l'aide d'une solution aqueuse dont le pH a été ajusté à l'aide d'une base, d'un acide ou d'un mélange acide/base. L'acide et/ou la base peuvent être inorganique ou organique.

Ces deux opérations peuvent aussi être effectuées après application de la composition.

Il est également possible de moduler la cinétique de polymérisation par voie anionique en pré-imprégnant les matières kératiniques à l'aide d'un agent nucléophile. L'agent nucléophile peut être utilisé pur, en solution, sous forme d'une émulsion, ou être encapsulé.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

De préférence, l'agent nucléophile est l'eau. Cette eau peut être apportée par une humidification préalable. Les agents nucléophiles particulièrement préférés sont les ions hydroxyles, notamment ceux présents dans l'eau.

Il est également possible, afin de moduler la cinétique de réaction, de préalablement humidifier la fibre à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant la fibre à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions peut être suivie ou non d'un rinçage. Ces compositions peuvent se présenter sous des formes diverses, telles que de lotion, de spray, de mousse et être appliquées sous forme de shampooing ou d'après-shampooing.

Le procédé peut comprendre une étape d'application sur les matières kératiniques d'au moins un polymère non siliconé tel que défini ci-dessus et une étape d'application sur les matières kératiniques d'au moins un monomère électrophile, l'ordre des étapes étant indifférent.

Dans un mode de réalisation particulier, l'application du ou des polymères non siliconés est réalisée avant l'application du ou des monomères électrophiles.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence de 20 à 80°C, ce qui n'empêche pas de terminer l'application par un séchage au casque, un brushing ou passage au fer plat ou à friser.

Les exemples qui suivent sont destinés à illustrer l'invention, sans toutefois présenter un caractère limitatif.

Dans les exemples, les pourcentages sont exprimés en pourcentage en poids de matière active.

### Exemple 1

On a préparé la composition suivante :

| | |
|---|---|
| 2-cyanoacrylate de n-octyle (1) | 60% |
| Polymère acrylique/acrylate d'éthyle/acrylonitrile commercialisé sous la dénomination HYSTRECH V-29 par la société Novéon | 39% |
| monoéthanolamine | 1% |

| | |
|---|---|
| (1) RITE LOK CON895, commercialisé par la société CHEMENCE | |

La force maximale de décollement, mesurée comme indiquée ci-dessus est de 4 N, et l'énergie de séparation est de 220 µJ.

La composition de l'invention permet, après plusieurs shampooings, de maintenir la douceur, la brillance et le maintien de la chevelure et ceci sans qu'il soit nécessaire d'appliquer à nouveau la composition.

### Exemple 2

On a préparé la composition suivante :

| | |
|---|---|
| methylheptylcyanoacrylate (1) | 60% |
| Polymère acrylique/acrylate d'éthyle/acrylonitrile commercialisé sous la dénomination HYSTRECH V-29 par la société Novéon | 39% |
| monoéthanolamine | 1% |

| | |
|---|---|
| (1) commercialisé par la société CHEMENCE | |

### Exemple 3

On a préparé la composition suivante :

| | |
|---|---|
| ethoxyethylcyanoacrylate (1) | 60% |
| Polymère acrylique/acrylate d'éthyle/acrylonitrile commercialisé sous la dénomination HYSTRECH V-29 par la société Novéon | 39% |
| monoéthanolamine | 1% |

| | |
|---|---|
| (1) EO 460 commercialisé par la société Tong Shen | |

### Exemple 4

On a préparé la composition suivante :

| | |
|---|---|
| butylcyanoacrylate (1) | 60% |
| Polymère acrylique/acrylate d'éthyle/acrylonitrile commercialisé sous la dénomination HYSTRECH V-29 par la société Novéon | 39% |
| monoéthanolamine | 1% |

| | |
|---|---|
| (1) B 60 commercialisé par la société Tong Shen | |

### Exemple 5

On a préparé la composition suivante :

| | |
|---|---|
| ethylhexylcyanoacrylate (1) | 60% |
| Polymère acrylique/acrylate d'éthyle/acrylonitrile commercialisé sous la dénomination HYSTRECH V-29 par la société Novéon | 39% |
| monoéthanolamine | 1% |

| | |
|---|---|
| (1) O-60 commercialisé par la société Tong Shen | |

### Exemple 6

On a préparé la composition suivante :

| | |
|---|---|
| methylheptylcyanoacrylate (1) | 54% |
| Ethylhexylcyanoacrylate (2) | 6% |
| Polymère acrylique/acrylate d'éthyle/acrylonitrile commercialisé sous la dénomination HYSTRECH V-29 par la société Novéon | 39% |
| monoéthanolamine | 1 % |

| | |
|---|---|
| (1) commercialisé par la société Chemence | |
| (2) O-60 commercialisé par la société Tong Shen | |

### Exemple 7

On a préparé la composition suivante :

| | |
|---|---|
| methylheptylcyanoacrylate (1) | 42% |
| butylcyanoacrylate (2) | 18% |
| Polymère acrylique/acrylate d'éthyle/acrylonitrile commercialisé sous la dénomination HYSTRECH V-29 par la société Novéon | 39% |
| monoéthanolamine | 1% |

| | |
|---|---|
| (1) commercialisé par la société Chemence | |
| (2) B-60 commercialisé par la société Tong Shen | |

## Revendications

1. Utilisation pour le traitement cosmétique des matières kératiniques, et notamment des fibres kératiniques telles que les cheveux, d'une composition comprenant, dans un milieu cosmétiquement acceptable, au moins un monomère électrophile, et au moins polymère non siliconé, le polymère non siliconé étant choisi de façon à ce que la composition donne après séchage un film présentant une force maximale de décollement supérieure à 1 Newton.

2. Utilisation selon la revendication 1, **caractérisée en ce que** le polymère non siliconé est choisi parmi les polymères fixants non siliconés.

3. Utilisation selon la revendication 2, **caractérisée en ce que** le polymère fixant non siliconé est choisi parmi les polymères fixants non siliconés cationiques, anioniques, amphotères, non ioniques et leurs mélanges.

4. Utilisation selon la revendication 3, **caractérisée en ce que** le polymère fixant non siliconé cationique est choisi parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

5. Utilisation selon la revendication 3, **caractérisée en ce que** le polymère fixant non siliconé anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques et les polyuréthanes anioniques.

6. Utilisation selon la revendication 3, **caractérisée en ce que** le polymère fixant non siliconé amphotère est choisi parmi les copolymères à motifs vinyliques acides et à motifs vinyliques basiques, les polyaminoamides réticulés et acylés, les polymères à motifs zwittérioniques, les polymères dérivés du chitosane, les copolymères alkyl(C₁-C₅)vinyléther/anhydride maléique modifiés et les polyuréthanes amphotères.

7. Utilisation selon la revendication 3, **caractérisée en ce que** le polymère fixant non siliconé non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame et les polyuréthanes non ioniques.

8. Utilisation selon l'une quelconque des revendications précédentes, **caractérisée en ce que** le ou les monomères électrophiles répondent à la formule : 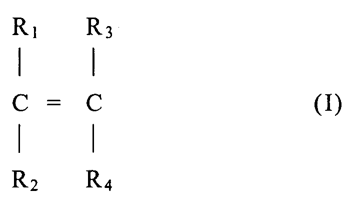 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, - S(R)₂⁺, -SH₂⁺, -NH₃⁺, -NO₂, -SO₂R, -C≡N, -COOH, -COOR, -COSR, - CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁- C₄, les groupements aryle et aryloxy,
R désigne un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un radical alkyle en C₁-C₁₀.

9. Utilisation selon la revendication 8, **caractérisée en ce que** le ou les monomères électrophiles sont choisis parmi les composés de formule : 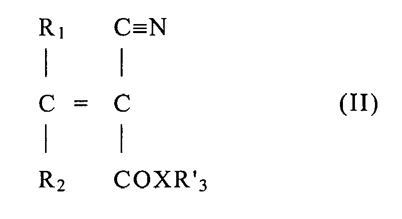 dans laquelle
X désigne NH, S, O,
R₁ et R₂ sont tels que définis dans la revendication 6,
R'₃ désigne un atome d'hydrogène ou un radical R tel que défini dans la revendication 6.

10. Utilisation selon la revendication 9, **caractérisée en ce que** le ou les monomères sont choisis parmi les 2-cyanoacrylates de polyfluoroalkyle en C₁-C₂₀, les cyanoacrylates d'alkyle en (C₁-C₁₀) ou d'(alcoxy en C₁-C₄)(alkyle en C₁-C₁₀).

11. Utilisation selon la revendication 10, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

12. Utilisation selon la revendication 9, **caractérisée en ce que** le ou les monomères électrophiles répondent à la formule (III) : 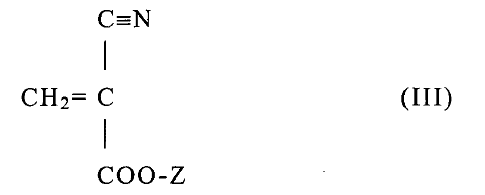 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

13. Utilisation selon l'une quelconque des revendications précédentes, **caractérisée en ce que** le monomère est présent dans la composition à des teneurs comprises entre 0,001 et 80% en poids, de préférence entre 0,1 et 40%, et encore de préférence entre 1 et 20% en poids du poids total de la composition.

14. Utilisation selon l'une quelconque des revendications précédentes, **caractérisée en ce que** les monomères sont fixés de façon covalente sur des supports tels que des polymères, des oligomères ou des dendrimères.

15. Utilisation selon l'une quelconque des revendications précédentes, **caractérisée en ce que** le milieu cosmétiquement acceptable est anhydre.

16. Utilisation selon la revendication 15, **caractérisé en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

17. Utilisation d'une composition selon l'une quelconque des revendications précédentes, **caractérisée en ce que** la composition comprend des inhibiteurs de polymérisation.

18. Utilisation selon la revendication 17, **caractérisée en ce que** les inhibiteurs sont des inhibiteurs de polymérisation anioniques et/ou radicalaires.

19. Utilisation selon la revendication 17, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

20. Utilisation selon l'une quelconque des revendications 17 à 19, **caractérisée en ce que** les inhibiteurs de polymérisation sont présents en des quantités allant de 10 ppm à 20%, de préférence allant de 10 ppm à 5%, et plus préférentiellement entre 10 ppm et 1% en poids du poids total de la composition.

21. Utilisation selon l'une quelconque des revendications précédentes, **caractérisée en ce que** polymère non siliconé est présent dans la composition à des teneurs comprises entre 0,05 et 99% en poids, de préférence entre 0,1 et 95%, et encore de préférence entre 0,2 et 30% en poids du poids total de la composition.

22. Utilisation selon l'une quelconque des revendications précédentes, **caractérisée en ce que** la composition comprend en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, cationiques, non ioniques ou amphotères, les polymères fixants ou non, les polyols, les protéines, les vitamines, les colorants directs ou d'oxydation, les agents nacrants, les gaz propulseurs, les épaississants minéraux ou organiques.

23. Utilisation selon la revendication 22, **caractérisée en ce que** l'agent est encapsulé.

24. Utilisation selon l'une quelconque des revendications précédentes, **caractérisée en ce que** les matières kératiniques sont les cheveux, les cils ou les ongles.

25. Utilisation selon l'une quelconque des revendications précédentes, **caractérisée en ce que** la composition est sous forme de lotion, de spray ou de mousse.

26. Composition cosmétique, **caractérisée en ce qu'**elle comprend, dans un milieu cosmétiquement acceptable, au moins un monomère électrophile défini dans l'une quelconque des revendications 6 à 11 et au moins un polymère fixant non siliconé.

27. Composition selon la revendication 26, **caractérisée en ce que** le polymère fixant non siliconé est choisi parmi les polymères fixants non siliconés cationiques, anioniques, amphotères, non ioniques et leurs mélanges.

28. Procédé de traitement des matières kératiniques, **caractérisé en ce qu'**il comprend une étape d'application sur les matières kératiniques d'au moins un polymère non siliconé tel que défini dans la revendication 1 et une étape d'application sur les matières kératiniques d'au moins un monomère électrophile.

29. Procédé selon la revendication 28, **caractérisée en ce que** l'application du ou des polymères non siliconés est réalisée avant l'application du ou des monomères électrophiles.

30. Procédé de traitement des matières kératiniques, **caractérisé en ce qu'**on applique sur les matières kératiniques une composition utilisée dans l'une quelconque des revendications 1 à 25, en présence d'un agent nucléophile.

31. Procédé selon la revendication 30, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PhNH⁻, pyridine, ArS⁻, R-C^{≡}C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle, Ar représentant un groupe aryle et R représentant un groupe aryle en C₁-C₁₀.

32. Procédé selon la revendication 30, **caractérisé en ce que** l'agent nucléophile est l'eau.

33. Procédé selon l'une quelconque des revendications 30 à 32, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

34. Procédé selon l'une quelconque des revendications 30 à 32, **caractérisé en ce que** les matières kératiniques sont pré-imprégnées à l'aide d'un agent nucléophile autre que l'eau.

35. Procédé selon l'une quelconque des revendications 30 à 32, **caractérisé en ce que** les matières kératiniques sont préalablement réduites avant application de la composition.

36. Procédé selon l'une des revendications 30 à 35, **caractérisé en ce que** l'application de la composition est suivie d'un rinçage.

37. Procédé selon l'une des revendications 30 à 36, **caractérisé en ce que** les matières kératiniques sont les cheveux.

38. Kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un polymère non siliconé tel que défini dans la revendication 1.
